## Europäisches Patentamt

## European Patent Office

## Office européen des brevets

(19)

(11) Veröffentlichungsnummer: **0 030 363**
**B1**

(12) **EUROPÄISCHE PATENTSCHRIFT**

(45) Veröffentlichungstag der Patentschrift:
**29.06.83**

(51) Int. Cl.³: **C 07 G 7/00, C 12 P 21/00**

(21) Anmeldenummer: **80107565.6**

(22) Anmeldetag: **03.12.80**

(54) **Verfahren zur Gewinnung von alpha-2-SB-Glykoprotein.**

(30) Priorität: **07.12.79 DE 2949407**

(43) Veröffentlichungstag der Anmeldung:
**17.06.81 Patentblatt 81/24**

(45) Bekanntmachung des Hinweises auf die Patenterteilung:
**29.06.83 Patentblatt 83/26**

(84) Benannte Vertragsstaaten:
**AT BE CH DE FR GB IT LI LU NL SE**

(56) Entgegenhaltungen:
DE-A-2 221 261
DE-A-2 256 910

,,Die Naturwissenschaften", Jahrgang 49, Heft 6,
1962, S. 133-134, H.E. Schultze *et al.*: ,,Über ein
*bisher unbekanntes saures* α-2-Glykoprotein".
,,Chemical abstracts" Bd. 76, Nr. 25, 19. Juni
1972, S. 266, 267, Nr. 151440p, Columbus, Ohio,
U.S.A., K. Warecka *et al.*: ,,Human brain-specific
α-2-glycoprotein purification by affinity chromatography and detection of new component. Localization in nervous cells".

(73) Patentinhaber: **BOEHRINGER MANNHEIM GMBH,
Sandhofer Strasse 112-132 Postfach 31 01 20,
D-6800 Mannheim 31-Waldhof (DE)**

(72) Erfinder: **Eberle, Walter, Eichenstrasse 10,
D-8131 Bernried (DE)**
Erfinder: **Albert, Winfried, Dr., Moosstrasse 10,
D-8121 Pähl (DE)**

(74) Vertreter: **Weickmann, Heinrich, Dipl.-Ing. et al,
Patentanwälte Dipl.-Ing. H. Weickmann Dipl.-Phys.Dr. K.
Fincke Dipl.-Ing. F.A. Weickmann Dipl.-Chem. B. Huber
Dr.-Ing. H. Liska Möhlstrasse 22,
D-8000 München 86 (DE)**

Verfahren zur Gewinnung von α-2-SB-Glykoprotein

Die Erfindung betrifft ein Verfahren zur Gewinnung von α-2-SB-Glykoprotein, welches auch als Fibronectin, kälteunlösliches Protein oder LETS-Protein bezeichnet wird, aus seinen physiologischen Lösungen, wie z.B. Plasma, Serum, Blut oder Organstroma.

α-2-SB-Glykoprotein ist eine Substanz, welche man im Blut und Organstroma findet. Das Serumprotein hat die elektrophoretische Beweglichkeit eines α-2-Globulins und einen Sedimentationskoeffizienten von 12 bis 14 S. Sein Molekulargewicht beträgt etwa 400 000 bis 440 000. Es besteht aus zwei Untereinheiten, welche durch Disulfidbrücken verbunden sind. Die Konzentration im Plasma von Gesunden liegt bei 300 bis 500 µg/ml. Eine nähere Beschreibung findet sich in „Nature", *275* (1978), 179.

α-2-SB-Glykoprotein ist ein Substrat des aktivierten Gerinnungsfaktors XIII, d.h. des Faktors XIIIa (Plasmatransglutaminase, Fibrinoligase). Es bindet Fibrinogen und Fibrin, vor allem in der Kälte. Da es während der Gerinnung mit Fibrin reagieren kann, findet man im Serum üblicherweise geringere Konzentrationen als im Plasma. Dem α-2-SB-Glykoprotein wurden auch Affinitäten zur Gelatine und Collagen zugeschrieben.

Die an die Zellmembran gebundene Form des α-2-SB-Glykoproteins liegt bei verschiedenen transformierten Zellen in wesentlich geringerer Dichte vor. Der veränderte Phänotyp dieser Zellen kann zu einem gewissen Grad durch die Adsorption von α-2-SB-Glykoprotein an die Zellmembran korrigiert werden (z.B. Wiederherstellung der Haftung an Kulturgefässen, Kontaktinhibition etc.).

Eine physiologische Eigenschaft des α-2-SB-Glykoproteins ist seine nichtimmunologische, opsonierende Wirkung. Z.B. wird die Aufnahme von partikulärem Material durch das retikuloendotheliale System (RES) gefördert. Es wurde eine direkte Korrelation zwischen dem α-2-SB-Glykoproteinspiegel im Plasma und der Aktivität des RES postuliert.

Bei bestimmten Erkrankungen, bei welchen es zu intravaskulären Gerinnungsstörungen kommt, tritt ein erhöhter Verbrauch von α-2-SB-Glykoprotein auf, wobei letzteres als unspezifisches Opsonin konsumiert wird. Der α-2-SB-Glykoproteinspiegel kann sich also im Verlaufe von gewissen Erkrankungen ändern. Erhöhte Werte treten z.B. bei Erkrankungen des Bindegewebes auf; bei metastasierenden Tumoren wurden häufig erhöhte Werte bei fortgeschrittenen Stadien beobachtet.

Man nimmt an, dass die Entfernung beschädigten autologen Gewebes und von zirkulierenden Bestandteilen, wie z.B. löslichem Fibrin, durch nichtimmunologische Opsonierung ein wichtiger physiologischer Vorgang ist. Aus diesem Grund kann eine beträchtliche Abnahme von α-2-SB-Glykoprotein zu Organversagen bei Schwerkranken führen. Durch Infusion von α-2-SB-Glykoprotein konnte ein positiver Effekt auf den Zustand von Patienten mit intravaskulären Gerinnungsstörungen, wie sie bei schweren Infektionen (Sepsis), bei Tumoren, schweren Verletzungen und nach Operationen vorkommen können, erzielt werden [„Science", *201* (1978), 622].

Vorraussetzung für eine gezielte Therapie ist jedoch eine ausreichende Verfügbarkeit von α-2-SB-Glykoprotein.

Die Affinitätschromatographie von α-2-SB-Glykoprotein an sepharosegebundener Gelatine wurde von Ruoslahti und Engvall [„Ann. of the New York Academy of Sciences", *312* (1978), 186] zur Isolierung dieses Proteins genutzt. Das Protein konnte mittels Harnstoff, Natriumthiocyanat, Natriumjodid, Äthylenglykol, spezifischen Antikörpern bzw. durch Collagenaseeinwirkung von der Gelatine enthaltenden Affinitätsmatrix eluiert werden. Nach „Biochem. J.", *175* (1978), 333 können verschiedene kationische Verbindungen (z.B. Arginin, Spermidin, Putrescin u.a.) dieses Protein von Gelatinesepharose eluieren. Diese Methoden haben gewisse Nachteile: Bei der Elution mit chaotropen Substanzen muss beispielsweise eine Dialyse angeschlossen werden. Nach der Entfernung dieser Substanzen kommt es dann zu Aggregationserscheinungen, wenn die Konzentration von α-2-SB-Glykoprotein 1 mg/ml übersteigt. Ein auf diese Weise hergestelltes Material lässt sich also nur schwer infundieren. Bei der Elution mit kationischen Substanzen wurde beobachtet, dass die Hämagglutinationsaktivität von α-2-SB-Glykoprotein inhibiert wurde. Eigene Untersuchungen ergaben darüber hinaus, dass die immunologischen Eigenschaften des α-2-SB-Glykoproteins nach Isolierung mittels kationischer Substanzen vermindert werden. In „Biochem. J.", *169* (1978), 55 wird ein Verfahren beschrieben, bei welchem α-2-SB-Glykoprotein durch Zusatz von 1 mol/l Kaliumbromid eluiert werden konnte. Dieses Verfahren leidet unter den gleichen Nachteilen, wie die oben erwähnten Methoden.

Aufgabe der Erfindung ist es daher, ein Verfahren zu schaffen, welches eine einfache Anreicherung bzw. Isolierung von α-2-SB-Glykoprotein ermöglicht, ohne dass hierbei Verluste in der immunologischen und physiologischen Aktivität auftreten.

Gelöst wird diese Aufgabe erfindungsgemäss durch ein Verfahren zur Gewinnung von α-2-SB-Glykoprotein aus seinen physiologischen Lösungen durch Bindung an ein an einem unlöslichen Träger immobilisiertes bindefähiges Protein, welches dadurch gekennzeichnet ist, dass anschliessend das trägerfixierte Protein mit Pufferlösung, pH 6 bis 8,5, gewaschen und danach das α-2-SB-Glykoprotein mit einer Pufferlösung von pH 9 oder höher eluiert und aus dem Eluat die gewünschte Substanz gewonnen wird.

Überraschenderweise wurde gefunden, dass sich unter den erfindungsgemässen pH-Bedingungen eine einfache Gewinnung des reinen Proteins ohne Veränderung seiner Eigenschaften, insbesondere ohne Aggregation, durchführen lässt.

Als bindefähiges Protein werden Gelatine, Collagen und Fibrin bevorzugt, die alleine oder in Mischung angewendet werden können. Ebenfalls geeignet sind jedoch auch andere bindefähige Proteine. Als Träger für das bindefähige Protein eignen sich die üblicherweise für die Immobilisierung von Proteinen verwendeten Trägermaterialien. Bevorzugt werden Träger auf Basis eines Kohlehydrats, wie Zellulose, Agarose oder dergleichen. Auch andere hydrophile organische oder anorganische Trägersubstanzen können verwendet werden. Typische Beispiele hierfür sind polymere Derivate der Acrylsäure oder Methacrylsäure, welche polare Substituenten, wie Hydroxylgruppen, Aminogruppen oder dergleichen tragen können.

Die Art der Bindung des bindefähigen Proteins am Träger ist nicht von Bedeutung, solange sie eine Auswaschung des immobilisierten bindefähigen Proteins vom Träger unter den Verfahrensbedingungen unmöglich macht. Bevorzugt werden kovalent fixierte Proteine. Zur kovalenten Trägerfixierung von Proteinen geeignete Verfahren sind in grösserer Anzahl dem Fachmann bekannt und brauchen hier nicht näher erläutert zu werden. Die Fixierung kann beispielsweise durch Aktivierung des Trägers, wie bromcyanaktivierte Kohlehydrate, durch Aktivierung des Proteins oder mittels difunktionellen Brückenbildnersubstanzen erfolgen.

Die physiologische α-2-SB-Glykoproteinlösung wird mit dem immobilisierten bindefähigen Protein kontaktiert und so lange in Kontakt gelassen, bis alles α-2-SB-Glykoprotein an den Träger gebundenvorliegt. Üblicherweise erfordert dies etwa 0,5 bis 2 h. Während der Kontaktierung wird zweckmässig die Mischung gerührt. Je nach der Art der physiologischen Lösung, ihrer Vorbehandlung, z.B. Citratplasma oder EDTA-Plasma (EDTA=Äthylendiaminotetraessigsäure), der Art des trägerfixierten bindefähigen Proteins und des Trägers kann jedoch die Dauer der Kontaktierung auch höher oder niedriger sein. Die Kontaktierungsdauer hängt ausserdem vom Verhältnis der Menge des eingesetzten immobilisierten bindefähigen Proteins und des in der Lösung vorhandenen Proteins ab.

Nach Beendigung der Kontaktierung bzw. Inkubation wird die Hauptmenge des ursprünglich in der Lösung vorhandenen Proteins mit einer Pufferlösung von pH 6 bis 8,5 ausgewaschen. Besonders geeignet ist der pH-Bereich von 7 bis 8, beste Ergebnisse werden bei pH 7,3 bis 7,7 erzielt. Die Pufferkonzentration kann in weiten Grenzen variiert werden. Gute Ergebnisse erhält man im allgemeinen bei Konzentrationen zwischen etwa 0,01 und etwa 0,3 mol/l. Zweckmässig wird der Pufferlösung zur Erhöhung der Ionenstärke ein neutrales Salz zugesetzt. Wird ein solches verwendet, so beträgt seine Konzentration vorzugsweise 0,5 bis 2 mol/l. Geeignete neutrale Salze sind beispielsweise Alkalichloride, wie Natriumchlorid, Kaliumchlorid, Lithiumchlorid usw., welche bevorzugt werden. Es können jedoch auch andere Salze starker Basen mit starken Säuren verwendet werden. Die Waschung der Trägersubstanz wird so lange durchgeführt, bis sich kein Protein mehr bei den angegebenen pH-Bedingungen auswaschen lässt.

Anschliessend erfolgt die Elution des α-2-SB-Glykoproteins vom Träger mit Hilfe einer Pufferlösung von pH 9 oder höher, vorzugsweise von pH 10 bis 12. Beste Ergebnisse werden bei pH-Werten zwischen 10,5 und 11,5 erzielt. Die so erhaltene, von Fremdprotein praktisch freie Lösung von α-2-SB-Glykoprotein kann nach Entfernung der Puffersubstanz durch Dialyse zur Trockne gebracht werden, beispielsweise durch Lyophilisation oder Eindampfen. Gemäss einer bevorzugten Ausführungsform verwendet man zur Elution eine flüchtige Puffersubstanz, so dass das erhaltene Eluat ohne vorherige Dialyse direkt der Lyophilisierung unterworfen werden kann.

Im Rahmen der Erfindung sind generell Puffersubstanzen geeignet, welche im angegebenen pH-Wertbereich zu puffern vermögen. Typische Beispiele für geeignete, nichtflüchtige Puffer sind Trissalzsäure-, Borat- Veronal-, Glycin/Natronlauge- und Carbonat puffer. Beispiele für geeignete flüchtige Puffer sind Triäthylammoniumbicarbonat-, Ammoniumbicarbonat/Kohlendioxid-, Ammoniumcarbonat-, Collidinacetat-, Pyridiniumacetat- und Cyclohexylaminopropansulfonatpuffer.

Das erfindungsgemässe Verfahren ist einfach durchzuführen und ermöglicht die Gewinnung eines reinen α-2-SB-Glykoproteins, dessen immunologische Aktivität und Bindeeigenschaften unverändert bleiben. Aufgrund der bekannten klebstoffartigen Eigenschaften des α-2-SB-Glykoproteins ist es als sehr überraschend anzusehen, dass es an einen unlöslichen Träger gebunden und von diesem unter milden Bedingungen wieder eluiert werden kann, ohne dass eine Aggregation auftritt.

Das folgende Beispiel erläutert die Erfindung weiter.

*Beispiel*

An bromcyanidaktivierte Sepharose (Handelsprodukt der Firma Pharmacia) wurde nach den Angaben des Herstellers 1 mg Gelatine/ml Sepharose kovalent gebunden.

20 l Humanplasma wurden mit 10 l Gelatinesepharose gemischt und über einen Zeitraum von 2 h unter Rühren inkubiert.

Anschliessend wurde die Sepharose über einer Glasfritte mit 0,05 mol/l Trissalzsäurepuffer, pH 7,5, welche 1 mol/l NaCl enthält, gewaschen, bis kein Protein mehr in der Waschlösung nachweisbar war.

Das gebundene α-2-SB-Glykoprotein wurde anschliessend mit 0,05 mol/l Cyclohexylaminopropansulfonsäure, welche durch Zugabe von 1 mol/l Natronlauge auf einen pH-Wert von 11,0 eingestellt wurde, eluiert. Die Gesamtausbeute betrug 4 g α-2-SB-Glykoprotein.

Das erhaltene Protein war immunelektrophoretisch reines α-2-SB-Glykoprotein. Dieses Protein wies nach der Elution volle immunologische Aktivität auf, seine Bindeeigenschaft zu den Affinitätsabsorbenzien nach Neutralisation blieb erhalten.

## Patentansprüche

1. Verfahren zur Gewinnung von α-2-SB-Glykoprotein aus seinen physiologischen Lösungen durch Bindung an ein an einem unlöslichen Träger immobilisiertes, bindefähiges Protein, dadurch gekennzeichnet, dass anschliessend das trägerfixierte Protein mit Pufferlösung, pH 6 bis 8,5, gewaschen und danach das α-2-SB-Glykoprotein mit einer Pufferlösung von pH 9 oder höher eluiert und aus dem Eluat gewonnen wird.

2. Verfahren nach Anspruch 1, dadurch gekennzeichnet, dass als bindefähiges Protein Gelatine, Collagen oder/und Fibrin verwendet wird.

3. Verfahren nach einem der beiden Ansprüche 1 oder 2, dadurch gekennzeichnet, dass man einen Träger auf Basis eines Kohlehydrats verwendet.

4. Verfahren nach einem der vorhergehenden Ansprüche, dadurch gekennzeichnet, dass man ein am Träger kovalent fixiertes, bindefähiges Protein verwendet.

5. Verfahren nach einem der vorhergehenden Ansprüche, dadurch gekennzeichnet, dass man das trägerfixierte Protein mit 0,01 bis 0,3 mol/l Puffer, pH 7 bis 8, welcher 0,5 bis 2 mol/l neutrales Salz enthält, wäscht.

6. Verfahren nach Anspruch 5, dadurch gekennzeichnet, dass man als neutrales Salz ein Alkalichlorid verwendet.

7. Verfahren nach einem der vorhergehenden Ansprüche, dadurch gekennzeichnet, dass man mit Puffer, pH 10 bis 12, eluiert.

8. Verfahren nach einem der vorhergehenden Ansprüche, dadurch gekennzeichnet, dass man mit einer flüchtigen Puffersubstanz eluiert.

9. Verfahren nach Anspruch 8, dadurch gekennzeichnet, dass man das Eluat direkt lyophilisiert.

## Claims

1. Process for the obtaining of α-2-SB-glycoprotein from its physiological solutions by binding on to a protein capable of binding, immobilised on an insoluble carrier, characterised in that the carrier-fixed protein is subsequently washed with buffer solution of pH 6 to 8.5, and thereafter the α-2-SB-glycoprotein is eluted with a buffer solution of pH 9 or higher and obtained from the eluate.

2. Process according to claim 1, characterised in that gelatine, collagen and/or fibrin is used as a protein capable of binding.

3. Process according to claim 1 or 2, characterised in that one uses a carrier based on a carbohydrate.

4. Process according to one of the preceding claims, characterised in that one uses a protein capable of binding, covalently fixed on to the carrier.

5. Process according to one of the preceding claims, characterised in that one washes the carrier-fixed protein with 0.01 to 0.3 mol/l buffer, pH 7 to 8, which contains 0.5 to 2 mol/l neutral salt.

6. Process according to claim 5, characterised in that one uses an alkali metal chloride as neutral salt.

7. Process according to one of the preceding claims, characterised in that one elutes with buffer, pH 10 to 12.

8. Process according to one of the preceding claims, characterised in that one elutes with a volatile buffer substance.

9. Process according to claim 8, characterised in that one lyophilises the eluate directly.

## Revendications

1. Procédé d'extraction de l'α-2-SB-glycoprotéine à partir de ses solutions physiologiques par liaison à une protéine capable de se lier, immobilisée sur un support insoluble, caractérisé en ce que la protéine fixée au support est ensuite lavée avec une solution-tampon, pH 6 à 8,5, et en ce que l'α-2-SB-glycoprotéine est ensuite éluée avec une solution-tampon à un pH de 9 ou supérieur et extraite de l'éluat.

2. Procédé suivant la revendication 1, caractérisé en ce qu'on utilise comme protéine capable de se lier de la gélatine, du collagène ou/et de la fibrine.

3. Procédé suivant l'une des revendications 1 ou 2, caractérisé en ce qu'on utilise un support à base d'un hydrate de carbone.

4. Procédé suivant l'une des revendications précédentes, caractérisé en ce qu'on utilise une protéine capable de se lier, fixée par covalence sur le support.

5. Procédé suivant l'une des revendications précédentes, caractérisé en ce qu'on lave la protéine fixée sur le support avec un tampon à 0,01 à 0,3 mol/l, pH 7 à 8, qui contient 0,5 à 2 mol/l de sel neutre.

6. Procédé suivant la revendication 5, caractérisé en ce qu'on utilise comme sel neutre un chlorure alcalin.

7. Procédé suivant l'une des revendications précédentes, caractérisé en ce qu'on élue avec un tampon à pH 10 à 12.

8. Procédé suivant l'une des revendications précédentes, caractérisé en ce qu'on élue avec une substance-tampon volatile.

9. Procédé suivant la revendication 8, caractérisé en ce qu'on lyophilise directement l'éluat.